(19) Europäisches Patentamt
European Patent Office
Office européen des brevets

(11) Veröffentlichungsnummer : **0 063 369**
**B1**

(12) **EUROPÄISCHE PATENTSCHRIFT**

(45) Veröffentlichungstag der Patentschrift : **25.07.84**

(51) Int. Cl.$^3$ : **C 07 J 1/00**

(21) Anmeldenummer : **82103221.6**

(22) Anmeldetag : **16.04.82**

(54) **Verfahren zur Herstellung von 17-alpha-Äthinyl-17-beta-trifluoracetoxygon-4-en-3-onderivaten.**

(30) Priorität : **16.04.81 HU 98781**

(43) Veröffentlichungstag der Anmeldung : **27.10.82 Patentblatt 82/43**

(45) Bekanntmachung des Hinweises auf die Patenterteilung : **25.07.84 Patentblatt 84/30**

(84) Benannte Vertragsstaaten : **CH DE FR GB IT LI NL SE**

(56) Entgegenhaltungen :
**JOURNAL OF ORGANIC CHEMISTRY, Band 41, Nr. 25, 10. Dezember 1976, Seiten 4036-4038, Washington DC (USA) G. ORTAR et al.: "Substitution and elimination reactions of steroid tertiary C-17 trifluoroacetates" Seite 4037, Beispiel 1**

(73) Patentinhaber : **RICHTER GEDEON VEGYESZETI GYAR R.T.**
**Gyömröl ut 19-21**
**H-1475 Budapest X (HU)**

(72) Erfinder : **Boòr, geb. Mezel, Anna**
**Mátyás király ut 4**
**H-1121 Budapest (HU)**
Erfinder : **Tòth, Jòzsef, Dr.**
**Alsò-Törökvész u. 10**
**Budapest II (HU)**
Erfinder : **Szén, Tamás, Dr.**
**Nagy Lajos király utja 151**
**H-1149 Budapest (HU)**
Erfinder : **Gábor, Lászlò**
**Gábor Aron u. 1/b**
**Budapest XVIII (HU)**
Erfinder : **Major, geb. Forstner, Piroska**
**Páskomliget u. 51/IX. 37**
**H-1156 Budapest (HU)**
Erfinder : **Holly, Sándor, Dr.**
**Városház u. 14**
**Budapest V (HU)**

(74) Vertreter : **Beszédes, Stephan G. Dr.**
**Münchener Strasse 80a Postfach 1168**
**D-8060 Dachau (DE)**

**EP 0 063 369 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung der neuen 17α-Äthinyl-17β-trifluoracetoxygon-4-en-3-on-derivate der allgemeinen Formel

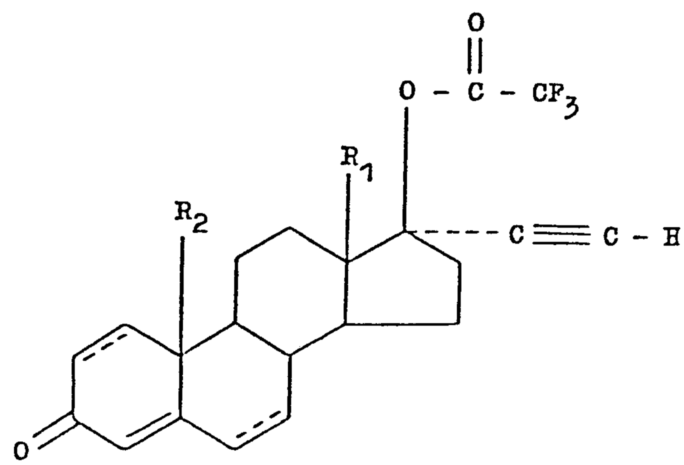

(I)

worin

$R_1$ für einen Methyl- oder Äthylrest steht,

$R_2$ Wasserstoff oder einen Methylrest bedeutet und

die gestrichelten Linien (---) gegebenenfalls vorliegende weitere chemische Bindungen darstellen.

Diese neuen 17α-Äthinyl-17β-trifluoracetoxygon-4-en-3-on-derivate sind Gegenstand der den ungarischen Patentanmeldungen mit den Aktenzeichen 989/81 und 987/81 vom 16. April 1981 entsprechenden gleichzeitig eingereichten europäischen Patentanmeldung EP-A-82103220.8 (63368).

Es wurde bereits die Trifluoracetylgruppe zum vorübergehenden Schutz von in der 11β-, 17α- und 21-Stellung befindlichen Hydroxygruppen bei bestimmten Steroiden mit Pregnangerüst verwendet (deutsche Auslegeschrift 2 144 405, US-Patentschrift 2 854 465 und britische Patentschrift 1 185 344), die Ester der 17α-Äthinyl-17β-hydroxygonane mit Trifluoressigsäure wurden jedoch bisher noch nicht beschrieben.

Ferner ist es aus dem Schrifttum bekannt, daß die Ester der Trifluoressigsäure gegen alkalische Hydrolyse sehr empfindlich sind und sich deshalb bei ihrer Isolierung aus dem wäßrigen Medium beziehungsweise beim Trocknen des wasserhaltigen Produktes zersetzen (Advances in Carbohydrate Chemistry 16 [1961], 60 bis 61 ; Chemical Reviews 55 [1955], 816 bis 817).

Der Erfindung liegt die Aufgabe zugrunde, ein vorteilhaftes Verfahren zur Herstellung der neuen 17α-Äthinyl-17β-trifluoracetoxygon-4-en-3-onderivate der allgemeinen Formel I zu schaffen.

Das Obige wurde überraschenderweise durch die Erfindung erreicht.

Es wurde nämlich nun überraschenderweise festgestellt, daß, obwohl die 17β-Hydroxygruppe der 17α-Äthinyl-17β-hydroxysteroide sterisch behindert ist und ihre Veresterung daher nur schwierig oder überhaupt nicht verwirklicht werden kann, die Umsetzung der im folgenden festgelegten 17α-Äthinyl-17β-hydroxygon-4-en-3-onderivate mit dem Säureanhydrid der Trifluoressigsäure schon bei 0 °C und einem geringen Überschuß des Trifluoressigsäureanhydrides bereits innerhalb weniger Minuten abläuft. Ferner wurde im Gegensatz zu den Angaben im oben genannten Schrifttum überraschenderweise festgestellt, daß die neuen 17α-Äthinyl-17β-trifluoracetoxygon-4-en-3-onderivate der allgemeinen Formel I verhältnismäßig stabile Verbindungen sind. So sind sie zum Beispiel stabil genug, um bei der Aufarbeitung von mit Wasser mischbare Basen, zum Beispiel Pyridin oder Dimethylformamid, enthaltenden Reaktionsgemischen beim Eingießen der Reaktionsgemische in Wasser und Abtrennen des ausgeschiedenen 17β-Trifluoressigsäureesters nicht zersetzt zu werden.

Gegenstand der Erfindung ist daher ein Verfahren zur Herstellung von 17α-Äthinyl-17β-trifluoracetoxygon-4-en-3-onderivaten der allgemeinen Formel

(Siehe Formel, Seite 3 f.)

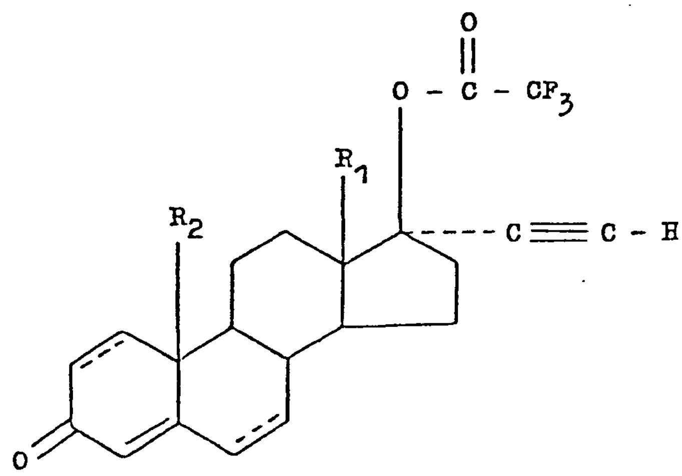

(I)

worin

$R_1$ für einen Methyl- oder Äthylrest steht,

$R_2$ Wasserstoff oder einen Methylrest bedeutet und

die gestrichelten Linien (---) gegebenenfalls vorliegende weitere chemische Bindungen darstellen, welches dadurch gekennzeichnet ist, daß 17α-Äthinyl-17β-hydroxygon-4-en-3-onderivate der allgemeinen Formel

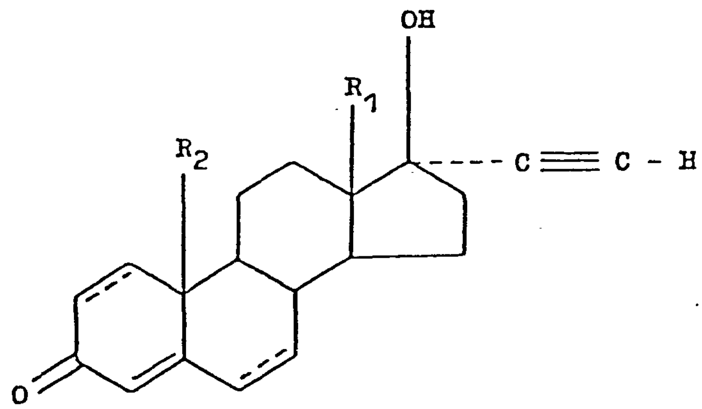

(II)

worin $R_1$ und $R_2$ sowie die gestrichelten Linien wie oben festgelegt sind, in wasserfreien Medien in Gegenwart von 1 oder mehr säurebindenden Mittel(n) mit mindestens der äquimolaren Menge Trifluoressigsäureanhydrid umgesetzt werden.

Zweckmäßig wird beziehungsweise werden als säurebindende[s] Mittel 1 oder mehr tertiäre Base(n) und/oder sonstige Substanz(en) von basischem Charakter verwendet.

Vorzugsweise wird beziehungsweise werden als als säurebindende[s] Mittel dienende[s] tertiäre[s] Amin(e) Pyridin, Triäthylamin und/oder N-Methylmorpholin verwendet.

Es ist auch bevorzugt, als als säurebindende[s] Mittel die dienende Substanz(en) von basischem Charakter Dimethylformamid und/oder Hexamethylphosphorsäuretriamid zu verwenden.

Zweckmäßig wird die Umsetzung bei Temperaturen von −50 bis +25 °C (Raumtemperatur) durchgeführt. Vorzugsweise werden Temperaturen von 0 bis 5 °C angewandt.

Als Lösungsmittel kann beziehungsweise können zweckmäßig das beziehungsweise die säurebindende(n) Mittel selbst, zum Beispiel Pyridin, Dimethylformamid und/oder Hexamethylphosphorsäuretriamid, verwendet werden. Erforderlichenfalls kann jedoch das Reaktionsgemisch auch mit inerten Lösungsmitteln, zum Beispiel Chloroform, Tetrahydrofuran, Dioxan und/oder Aceton, verdünnt werden.

Die im erfindungsgemäßen Verfahren als Ausgangsstoffe zu verwendenden 17α-Äthinyl-17β-hydroxygon-4-en-3-onderivate der allgemeinen Formel II können aus entweder durch Abbau der Seitenkette von Sterinen (zum Beispiel Cholesterin, Sitosterin oder Stigmasterin) oder durch Totalsynthese erhaltenen 17-Ketosteroiden durch Äthinylieren in an sich bekannter Weise erhalten worden sein.

Die 17α-Äthinyl-17β-trifluoracetoxygon-4-en-3-onderivate der allgemeinen Formel I zeigen eine pharmakologische, insbesondere empfängnisverhütende, Wirksamkeit, sie sind jedoch auch wertvolle Ausgangsstoffe zur Herstellung der in der den ungarischen Patentanmeldungen mit den Aktenzeichen 989/81 und 987/81 vom 16. April 1981 entsprechenden gleichzeitig eingereichten europäischen Patentanmeldung EP-A-82103220.8(63368) beschriebenen 17β-Formyloxy-, 17β-Acetyloxy- und 17β-Hydroxypregn-4-en-3,20-dionderivate mit wertvoller pharmakologischer, insbesondere progestativer, Wirkung (vergleiche The Merck Index. 9. Auflage, Nr. 4756) und für die Synthese von eine Corticoidwirkung

aufweisenden anderen Verbindungen mit Pregnangerüst. Als Beispiele für diese letzteren Verbindungen seien die folgenden genannt : Die Reichstein-S-Verbindung, über die Hydrocortison, Prednisolon und Triamcinolonacetonid in an sich bekannter Weise hergestellt werden können, sowie ferner das eine gestagene Wirkung aufweisende 17α-Hydroxyprogesteroncapronat. In den Synthesen dieser Verbindungen ist die 17β-Trifluoracetoxygruppe als austretende Gruppe sehr vorteilhaft, weswegen ausgehend von den 17α-Äthinyl-17β-trifluoracetoxygon-4-en-3-onderivaten die die entsprechende räumliche Stellung aufweisende 17α-Acyloxy-20-oxopregnanstruktur mit sehr guter Ausbeute ausgebildet werden kann.

Die Erfindung wird an Hand der folgenden Beispiele näher erläutert.

Beispiel 1

3,10 g (0,01 Mol) 17α-Äthinyl-17β-hydroxy-androst-4-en-3-on werden in 15 ml wasserfreiem Pyridin gelöst. Die Lösung wird auf 0 °C gekühlt und unter Kühlung tropfenweise mit 2,09 ml (0,015' Mol) Trifluoressigsäureanhydrid versetzt. Das Gemisch wird bei 0-5 °C 10 Minuten lang gerührt und dann in 310 ml Wasser gegossen. Das sich abscheidende kristalline Produkt wird abfiltriert, mit Wasser gewaschen und dann getrocknet. 3,55 g 17α-Äthinyl-17β-trifluoracetoxy-androst-4-en-3-on werden erhalten (99,3 % der theoretischen Ausbeute). Schmelzpunkt : 142-144 °C.

IR : 1 664 (3—C=O), 1 785-1 215 (—O—CO—$CF_3$), 1 612 (C=C), 1 150 (—$CF_3$), 3 300, 3 235, 2 120 (—C≡CH) $cm^{-1}$.

Elementaranalyse für Fluor : berechnet : 13,95 % ; gefunden : 13,76 %.

Beispiel 2

3,08 g (0,01 Mol) 17α-Äthinyl-17β-hydroxy-androsta-1,4-dien-3-on werden in 15,4 ml Pyridin gelöst. Zu der Lösung werden bei 0-5 °C unter Kühlung 2,09 ml (0,015 Mol) Trifluoressigsäureanhydrid gegeben. Nach Absetzen der Kühlung wird das Gemisch noch 15 Minuten lang gerührt und dann in 308 ml Wasser gegossen. Das ausgeschiedene Produkt wird abfiltriert und getrocknet. 3,49 g (98,0 %) 17α-Äthinyl-17β-trifluor-acetoxy-androsta-1,4-dien-3-on werden erhalten, das bei 149-152 °C schmilzt.

IR : 1 657 (3—C=O), 1 780, 1 215 (—O—CO—$CF_3$), 1 619, 1 601 (C=C), 1 165 (—$CF_3$), 3 220, 2 110 (—C≡CH) $cm^{-1}$.

Elementaranalyse für Fluor : berechnet : 14,03 % ; gefunden : 13,91 %.

Beispiel 3

Zu der Lösung von 3,06 g (0,01 Mol) 17α-Äthinyl-17β-hydroxy-androsta-1,4,6-trien-3-on in 30,6 ml Pyridin werden bei 0-5 °C unter Eiskühlung 2,09 ml (0,015 Mol) Trifluoressigsäureanhydrid gegeben. Das Reaktionsgemisch wird bei 0-5 °C eine Stunde lang gerührt und dann in 306 ml Wasser gegossen. Das ausgeschiedene kristalline Produkt wird abfiltriert und getrocknet. 3,47 g (98,0 %) 17α-Äthinyl-17β-trifluoracetoxy-androsta-1,4,6-trien-3-on werden erhalten. Das Produkt schmilzt bei 132-134 °C.

IR : 1 785, 1 216 (—O—CO—$CF_3$), 1 645 (3—C=O), 1 610, 1 575 (C=C), 1 150 (—$CF_3$), 3 265, 2 120 (—C≡CH) $cm^{-1}$.

Elementaranalyse für Fluor : berechnet : 14,09 % ; gefunden : 14,20 %.

Beispiel 4

Zu der Lösung von 3,10 g (0,01 Mol) 17α-Äthinyl-17β-hydroxy-18-methyl-östr-4-en-3-on in 30 ml Pyridin werden bei 0-5 °C unter Kühlung 2,09 ml (0,015 Mol) Trifluoressigsäureanhydrid gegeben. Das Reaktionsgemisch wird bei der angegebenen Temperatur 30 Minuten lang gerührt und dann in 450 ml Wasser gegossen. Die ausgeschiedene kristalline Substanz wird abfiltriert und getrocknet. 3,45 g (96,5 %) 17α-Äthinyl-17β-trifluoracetoxy-18-methyl-östr-4-en-3-on werden erhalten. Schmelzpunkt : 192-194 °C.

IR : 1 782, 1 216 (—O—CO—$CF_3$), 1 647 (3—C=O), 1 609 (C=C), 1 150 (—$CF_3$), 3 220, 2 110 (—C≡CH) $cm^{-1}$.

Elementaranalyse für Fluor : berechnet : 13,95 % ; gefunden : 13,78 %.

Beispiel 5

2,96 g (0,01 Mol) 17α-Äthinyl-17β-hydroxy-östr-4-en-3-on werden in 29,6 ml Pyridin gelöst. Zu der auf 0 °C gekühlten Lösung werden tropfenweise 2,09 ml (0,015 Mol) Trifluoressigsäureanhydrid gegeben. Das Reaktionsgemisch wird 30 Minuten lang gerührt und dann in 300 ml Wasser gegossen. Die ausgeschiedene kristalline Substanz wird abfiltriert und getrocknet. 3,27 g (95,0 %) 17α-Äthinyl-17β-trifluoracetoxy-östr-4-en-3-on werden erhalten. Das Produkt schmilzt bei 145-146 °C

IR : 1 785, 1 220 (—O—CO—$CF_3$), 1 650, 1 609 (3—C=O), 1 155 (—$CF_3$), 3 210, 2 105 (—C≡CH) $cm^{-1}$.

Elementaranalyse für Fluor : berechnet : 14,84 % ; gefunden : 14,72 %.

**Ansprüche**

1. Verfahren zur Herstellung von 17α-Äthinyl-17β-trifluoracetoxygon-4-en-3-onderivaten der allgemeinen Formel

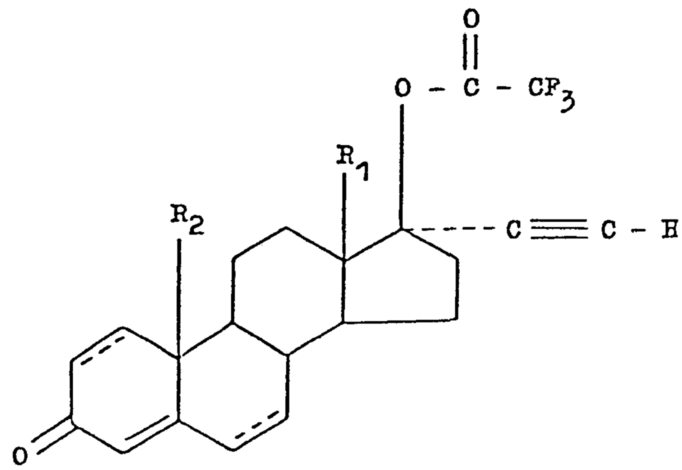

(I)

worin

$R_1$ für einen Methyl- oder Äthylrest steht,

$R_2$ Wasserstoff oder einen Methylrest bedeutet und

die gestrichelten Linien (-----) gegebenenfalls vorliegende weitere chemische Bindungen darstellen, dadurch gekennzeichnet, daß man 17α-Äthinyl-17β-hydroxygon-4-en-3-onderivate der allgemeinen Formel

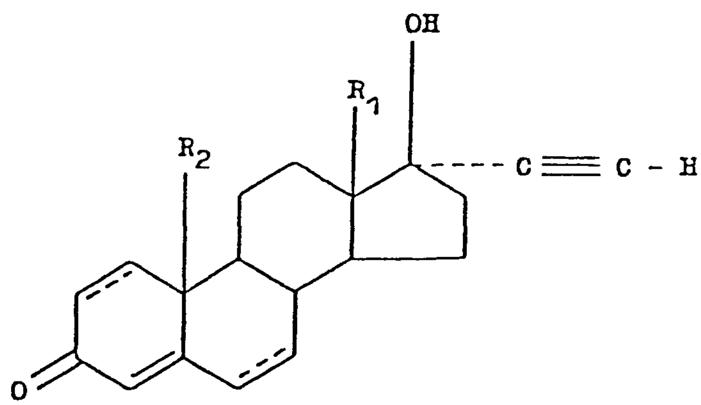

(II)

worin $R_1$ und $R_2$ sowie die gestrichelten Linien wie oben festgelegt sind, in wasserfreien Medien in Gegenwart von 1 oder mehr säurebindenden Mittel(n) mit mindestens der äquimolaren Menge Trifluoressigsäureanhydrid umsetzt.

2. Verfahren nach Anspruch 1, dadurch gekennzeichnet, daß man als säurebindende[s] Mittel 1 oder mehr tertiäre Base(n) und/oder sonstige Substanz(en) von basischem Charakter verwendet.

3. Verfahren nach Anspruch 1 oder 2, dadurch gekennzeichnet, daß man als als säurebindende[s] Mittel dienende[s] tertiäre[s] Amin(e) Pyridin, Triäthylamin und/oder N-Methylmorpholin verwendet.

4. Verfahren nach Anspruch 1 bis 3, dadurch gekennzeichnet, daß man als als säurebindende[s] Mittel dienende Substanz(en) von basischem Charakter Dimethylformamid und/oder Hexamethylphosphorsäuretriamid verwendet.

5. Verfahren nach Anspruch 1 bis 4, dadurch gekennzeichnet, daß man die Umsetzung bei Temperaturen von − 50 bis + 25 °C durchführt.

6. Verfahren nach Anspruch 1 bis 5, dadurch gekennzeichnet, daß man die Umsetzung bei Temperaturen von 0 bis 5 °C durchführt.

**Claims**

1. Process for preparing 17α-ethinyl-17β-trifluoroacetoxygon-4-ene-3-one derivatives having the general formula

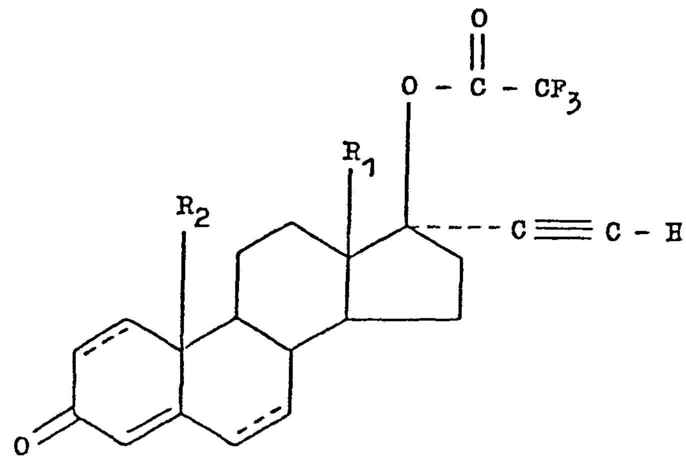

(I)

wherein

$R_1$ represents a methyl or ethyl radical,

$R_2$ means hydrogen or a methyl radical and

the dotted lines (-----) represent further chemical bonds optionally present,

characterized in that one reacts 17α-ethinyl-17β-hydroxygon-4-ene-3-one derivatives having the general formula

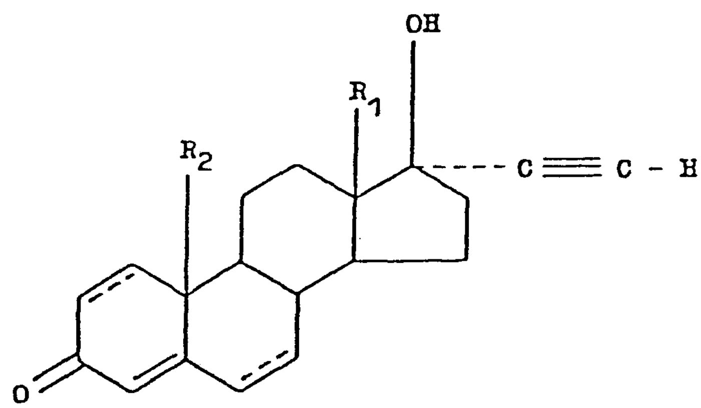

(II)

wherein $R_1$ and $R_2$ as well as the dotted lines are as above defined under anhydrous conditions in the presence of 1 or more acid-binding agent(s) with at least the equimolar amount of trifluoroacetic anhydride.

2. Process according to claim 1, characterized in that one uses as [an] acid-binding agent(s) 1 or more tertiary base(s) and/or other substance(s) of basic character.

3. Process according to claim 1 or 2, characterized in that one uses as [a] tertiary amine(s) serving as [an] acid-binding agent(s) pyridine, triethylamine and/or N-methylmorpholine.

4. Process according to claims 1 to 3, characterized in that one uses as substance(s) of basic character serving as [an] acid-binding agent(s) dimethylformamide and/or hexamethylphosphoric acid triamide.

5. Process according to claims 1 to 4, characterized in that one carries out the reaction at temperatures of from − 50 to + 25 °C.

6. Process according to claims 1 to 5, characterized in that one carries out the reaction at temperatures of from 0 to 5 °C.

**Revendications**

1. Procédé de préparation de dérivés 17α-éthynyl-17βtrifluoracétoxygon-4-ène-3-one de formule générale I

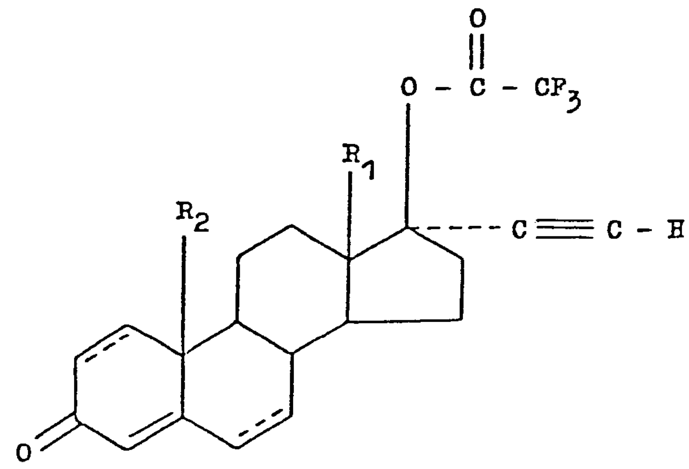

(I)

dans laquelle

$R_1$ représente un radical méthyle ou éthyle,

$R_2$ représente l'hydrogène ou un radical méthyle, et

les tiretés (----) représentent des liaisons chimiques supplémentaires existant éventuellement, caractérisé par le fait que l'on fait réagir dans des milieux aqueux, en présence d'un ou plusieurs accepteurs d'acide, des dérivés 17α-éthynyl-17β-hydroxygon-4-ène-3-one de formule générale II :

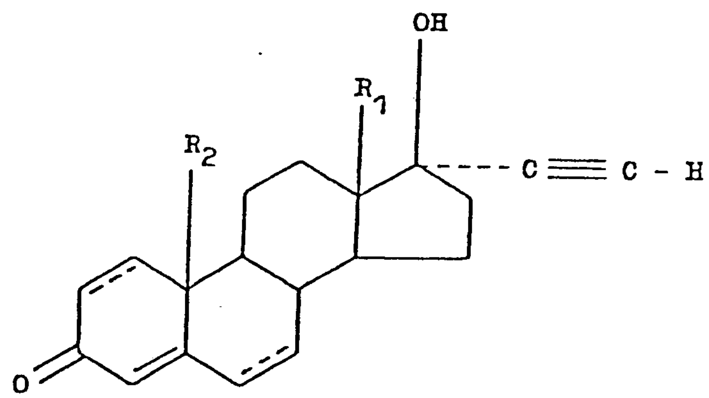

(II)

dans laquelle $R_1$ et $R_2$ ainsi que les tiretés sont tels que définis ci-dessus, sur au moins la quantité équimolaire d'anhydride trifluoracétique.

2. Procédé selon la revendication 1, caractérisé par le fait que l'on utilise, comme accepteurs d'acide, une ou plusieurs bases tertiaires et/ou autres substances à caractère basique.

3. Procédé selon l'une des revendications 1 et 2, caractérisé par le fait que comme amines tertiaires servant d'accepteurs d'acide, on utilise la pyridine, la triéthylamine et/ou la N-méthylmorpholine.

4. Procédé selon les revendications 1 à 3, caractérisé par le fait que comme substances à caractère basique servant d'accepteurs d'acide, on utilise le diméthylformamide et/ou l'hexaméthylphosphorotriamide.

5. Procédé selon les revendications 1 à 4, caractérisé par le fait que l'on conduit la réaction à des températures de − 50 à + 25 °C.

6. Procédé selon les revendications 1 à 5, caractérisé par le fait que l'on conduit la réaction à des températures de 0 à 5 °C.